# EUROPEAN PATENT SPECIFICATION

(11) **EP 2 322 244 B1**
(45) Date of publication and mention of the grant of the patent: **21.11.2012**
(21) Application number: 10192206.0
(22) Date of filing: 09.09.2005
(51) Int. Cl.: A61P 33/06, A61K 9/20, A61K 31/357, A61K 31/505, A61K 31/4965

(54) **Pharmaceutical composition comprising artesunate, sulfamethoxypyrazine and pyrimethamine suitable for the treatment of malaria within one day, in the form of a tablet**
Pharmazeutische Zusammensetzung enthaltend Artesunat, Sulfamethoxypyrazin und Pyrimethamin, die zur Behandlung von Malaria innerhalb eines Tages geeignet ist, in Form einer Tablette
Composition pharmaceutique à base d'artesunate, de sulfaméthoxypyrazine et de pyriméthamine permettant le traitement du palludisme en un jour, sous forme de comprimé

(43) Date of publication of application: 18.05.2011
(62) Divisional of application: 05795966.0
(73) Proprietor: Dafra Pharma N.V., 2300 Turnhout (BE)
(72) Inventor: Platteeuw, Johannes, 5283 JH, Boxtel (NL); Jansen, Frans Herwig, 2360, Oud-Turnhout (BE)
(74) Representative: van Kooij, Adriaan

(56) References cited:
- LUXEMBURGER C ET AL: "Single day mefloquine-artesunate combination in the treatment of multi-drug resistant falciparum malaria", TRANSACTIONS OF THE ROYAL SOCIETY OF TROPICAL MEDICINE AND HYGIENE, ELSEVIER, GB, vol. 88, no. 2, 1 March 1994 (1994-03-01), pages 213-217, XP023097868, ELSEVIER, GB ISSN: 0035-9203, DOI: 10.1016/0035-9203(94)90303-4 [retrieved on 1994-03-01]
- DAO ET AL: "Fatty food does not alter blood mefloquine concentrations in the treatment of falciparum malaria", TRANSACTIONS OF THE ROYAL SOCIETY OF TROPICAL MEDICINE AND HYGIENE, vol. 99, no. 12, 6 September 2005 (2005-09-06), pages 927-931, XP005105637, ELSEVIER, GB ISSN: 0035-9203, DOI: 10.1016/J.TRSTMH.2005.04.016
- WIJEYARATNE P M ET AL: "Therapeutic efficacy of antimalarial drugs along the eastern Indo-Nepal border: a cross-border collaborative study", TRANSACTIONS OF THE ROYAL SOCIETY OF TROPICAL MEDICINE AND HYGIENE, vol. 99, no. 6, 1 June 2005 (2005-06-01), pages 423-429, XP004852109, ELSEVIER, GB ISSN: 0035-9203, DOI: 10.1016/J.TRSTMH.2004.09.011
- ANONYMOUS: "Co-Arinate", DAFRA PHARMA 2004, [Online] 8 March 2005 (2005-03-08), XP002372153, Retrieved from the Internet: URL:http://web.archive.org/web/20050308092 738/http://www.dafra.be/products.php?id=68 > [retrieved on 2006-03-15]
- "Parallel sessions - abstracts of presentations", ACTA TROPICA, ELSEVIER SCIENCE BV., AMSTERDAM, NL, vol. 95, 2005, pages 1-506, XP005041020, ISSN: 0001-706X
- Anonymous: "Rapid Assessment Report- Uganda", Malaria Operational Plans (MOPs) , 13 August 2005 (2005-08-13), pages 1-25, XP002626032, Retrieved from the Internet: URL:http://www.fightingmalaria.gov/countri es/mops/assessments/uganda_assessment.pdf [retrieved on 2011-03-01]
- BIAGINI ET AL: "Current drug development portfolio for antimalarial therapies", CURRENT OPINION IN PHARMACOLOGY, vol. 5, no. 5, 9 August 2005 (2005-08-09), pages 473-478, XP005059119, ELSEVIER SCIENCE PUBLISHERS, NL ISSN: 1471-4892, DOI: 10.1016/J.COPH.2005.05.004

## Description

The present invention relates to a pharmaceutical composition suitable for the treatment of malaria within one day and to the use thereof or its constituents for the treatment of malaria.

Drug resistant malaria is a serious clinical and public health problem. Malaria is caused by an infection with the parasite Plasmodium falciparum. This parasite has developed a versatile capacity of evading the effect of many known antimalarial agents either by genetic or by non-genetic methods.

It has been recognized since many years that artemisinin, a sesquiterpene lactone with a peroxide moiety, is a potent antimalarial agent. Artemisinin is usually isolated from Artemisia annua L and many derivatives of artemisinin are known such as artemether, arteether, dihydroartemisinin and artesunate with a similar antimalarial activity.

Artemisinin based monotherapy, a therapy using one active compound, has been extensively used in Asia and Africa in the ongoing battle against Malaria since the discovery of the molecule as an antimalarial agent.

A short treatment course of 3 days is sufficient to cure a patient but reoccurrence in this case is fairly common. Therefore, it was suggested to extend the therapy to 5 or even 7 days. This turned out to give good results and a five day treatment course, if fully completed, leads to cure rates of more than 90 % at follow up for 28 days.

Athough the administration of artemisinin or its derivatives can effectively combats a Plasmodium falciparum infection, one of the drawbacks of this monotherapy is that it takes a minimum of 5 days. A further drawback is that artemisinin or its derivatives are the last line of defense against malaria, making many people concerned about the possibility for development of resistance against these antimalarial agents.

Since a couple of years, in order to avoid the development of drug resistance by the malarial parasite Plasmodium falciparum, it has been recommended by the World Helath Organization (WHO) and other organisations like for example Médecins sans frontières (MSF) to combine an artemisinin monotherapy with one or more drugs, usually also antimalarial agents, having a different mode of action to slow down or to eliminate the development of resistance against these antimalarial agents and further to improve patient compliance by reducing the therapy duration.

Public as well as company based researchers have taken up this challenge and have come up with several possible combinations. Combination therapy exist in co-blisters or Fixed dose formulations of artemisinin or its derivates with a number of other known or unknown antimalarial compounds. Specific examples of these are combinations of artesunate with amodiaquine, of artemether with lumefantrine, of artesunate with mefloquine and suggested are artesunate with chlorproguanil/dapsone and dihydroartemisinin with piperaquine.

Ideally, these combinations should be cheap, easy to administer, safe for children and pregnant women, and the elimination half-lifes of the drugs should match to keep the synergy going for the complete therapeutic interval and the formulation should be stable in tropical conditions.

A promising combination appears to be a combination of artemisinin and two types of antifolate agents such as a dihydropteroate synthase (DHPS) inhibitor and a dihydrofolate reductase (DHFR) inhibitor. A DHPS-inhibitor works by competing with p-aminobenzoic acid for the reactive site on the DHPS-enzyme. A DHFR-inhibitor completely blocks the enzyme in the last step of the folic acid biosynthesis.

Sulfadrugs, like sulfadoxine, sulfamethoxazole, sulfalene and dapsone fall into the category of DHPS inhibitors, whilst the DHFR group is usually represented by trimethoprim, pyrimethamine and or (chlor)proguanil. Although technically speaking antifolate therapy is a combination therapy in itself, the general view is that since the target is the same metabolic pathway.

The application of antifolate therapy to malaria was started by Hoffman la Roche with the introduction of a fixed combination of sulfadoxine and pyrimethamine, better known as Fansidar. Shortly after that Farmitalia, introduced Metakelfin, which is a combination of sulfamethoxypyrazine also known as sulfalene (belonging to the DHPS group) with pyrimethamine (belonging to the DHFR group). However, since Fansidar was the first drug in this class of therapy and the presence of Roche in the marketplace was extensive, Metakelfin never reached the expected sales volume and Fansidar became the predominant antimalarian drug in the market.

Treatment with antifolates has shown reduced efficacy over the last few years, as malaria parasites are known to have developed genetic point mutations which make these drugs less effective. In the discussion about the increasing resistance of the malaria parasite to sulfadrugs, research on Fansidar became instrumental. Unfortunately this has resulted in many generalizations, eventually leading most people to believe that all sulfadrugs are equal.

The above combinations of artemisinin or its derivatives with the two different types of foliate inhibitors and specifically artesunate with sulfadoxine and pyrimethamine provide an effective treatment of malaria within three days. For this, these compounds are usually administered in the form of a tablet comprising artemisinin or its derivatives such as artesunate during three days and the foliate inhibitors sulfadoxine and pyrimethamine are given as a single dose during day 1 of the treatment.

However, there is still a need to further reduce the treatment time because of at least four reasons.

First, in order to avoid the occurrence of resistance to the antimalarial agents used, the treatment time should be as short as possible. This because the faster the Plasmodium falciparum parasites are cleared from the system, the less time remains for the parasite to develop drug resistance.

Second, a shorter duration of the treatment would make it more easier to completely finish the treatment. This further decreases the risk of developing resistant Plasmodium falciparum parasites.

Third, a shorter treatment protocol requiring less treatment days can be easier monitored by trained physicians and nurses and in addition allows for the treatment of more people in a local clinic in a shorter period of time.

Fourth, the shorter treatment protocol is more convenient for the patient not in the least because the period of suffering from the disease is reduced.

The above objects are met by the pharmaceutical composition according to the present invention providing a treatment of malaria in one day.

In Luxemburger et al, T Roy Soc Trop Med H, 1994 and Dao et al, T Roy Soc Trop Med H, 2005, artesunate tablets were administered with mefloquine. Treatment over three days was equivalent to one day treatment. According to Wijeyaratne et al, T Roy Soc Trop Med H, 2005, the three-day regimen of an artesunate and sulfadoxine/pyrimethamine combination was more efficacious than the single day regimen.

According to the present invention a pharmaceutical composition is provided comprising an antimalarial agent selected from the group consisting of artemether, arteether, artemisinin, dihydroartemisinin and artesunate; sulfamethoxypyrazine (SM); and a dihydrofolate reductase inhibitor and further comprising one or more pharmaceutical acceptable excipients, carriers and/or diluents.

Sulfamethoxypyrazine is a well-characterized sulfonamide drug belonging to the class of dihydropteroate synthase (DHPS) inhibitors and is chemically very close to sulfadoxine but biologically quite distinct with for example a shorter half-life and less plasma protein binding.

The efficacy of the sulfamethoxypyrazine-dihydrofolate reductase inhibitor combination, and specifically pyrimethamine, in treating Plasmodium falciparum malaria has been demonstrated in the past, although it has not been used widely as an antimalarial for over 10 years. The main problem is that the combination does not reduce the number of gametocytes. This leads to higher transmission rates and also within 28 days 10 to 20 % of the people suffer from a re-infection or recrudescence.

However, in combination with artemisinin, it not only increases the efficacy of the treatment, but, surprisingly, reduces the necessary treatment time to one day.

According to one preferred embodiment of the present invention the antimalarial agent is artesunate. Artesunate is preferred because of his stability and price.

According to another preferred embodiment, the dihydrofolate reductase inhibitor in pharmaceutical composition according to the present invention is pyrimethamine being the most active dihydrofolate reductase inhibitor, although other inhibitors can be contemplated such as preferably (chlor)proguanyl. However, the most preferred dihydrofolate reductase inhibitor remains pyrimethamine.

In the pharmaceutical composition according to the present invention the preferred ratio between the antimalarial agent and the sulfamethoxypyrazine is (50 to 150):(200 to 300), more preferably about 100:250, such as specifically 100:250.

Also preferred is a ratio between the sulfamethoxypyrazine and the dihydrofolate reductase inhibitor of (200 to 300):(10 to 15). In this case the more preferred ratio is about 250:12.5, such as specifically 250:12.5.

Regarding the ratio between the antimalarial agent and the dihydrofolate reductase inhibitor in the pharmaceutical composition according to the present invention the preferred ratio is (50 to 150):(10 to 15), more preferably about 100:12.5, such as specifically 100:12.5.

The pharmaceutical composition according to the present invention can be administered using the common routes of administration such as oral, rectal, parenteral or intramuscular administration routes, however the preferred route of administration is the oral route.

Also, the pharmaceutical composition according to the present invention can be formulated into any known doses form know in the pharmaceutical field such as formulated as a tablet, an injectable solution, a capsule, a granule, a suppository, a powder, a particulate, a micro particulate or a syrup, however, the preferred formulation is a tablet.

Although the specific amounts of active ingredients in the pharmaceutical composition according to the present invention in order to obtain an effective treatment of malaria depend on, amongst others, the condition of the patient, the age of the patient, the disease state, etc, which conditions are in general taken into account by a trained physician. The amounts of active ingredients usually are about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively, in case these active constituents form the pharmaceutical composition according to the present invention.

According to another aspect, the present invention relates to a pharmaceutical composition kit comprising two or three doses forms, wherein the pharmaceutical composition kit comprises an antimalarial agent selected from the group consisting of artemether, arteether, artemisinin, dihydroartemisinin and artesunate; sulfamethoxypyrazine; and a dihydrofolate reductase inhibitor comprised alone or in combination in the two or three doses forms.

This pharmaceutical composition kit provides to the patients suffering from malaria, the active ingredients in two or three doses forms, such as preferably tablets. In case of two doses forms, one doses form can for example comprise the antimalarial agent and sulfamethoxypyrazine and the other the dihydrofolate reductase inhibitor. An other example is wherein one doses form comprises the antimalarial agent and the other doses form comprises the sulfamethoxypyrazine and the dihydrofolate reductase inhibitor. Also a pharmaceutical composition kit can be envisaged comprising two dosage forms whereby one doses form comprises the antimalarial agent and the dihydrofolate reductase inhibitor and the other doses form comprises the sulfamethoxypyrazine. Of course, in case the pharmaceutical composition kit comprises three dosage forms, the active constituents are the single active ingredient of the individual doses form, thus one doses form comprising an antimalarial agent selected from the group consisting of artemether, arteether, artemisinin, dihydroartemisinin and artesunate, one doses form comprising sulfamethoxypyrazine and one doses form comprising the dihydrofolate reductase inhibitor.

The preferred antimalarial agent and dihydrofolate reductase inhibitor in the pharmaceutical composition kit according to the present invention are artesunate and pyrimethamine, respectively.

According to one preferred embodiment of the pharmaceutical composition kit according to the present invention the kit comprises a tablet comprising artesunate and a tablet comprising the combination sulfamethoxypyrazine and pyrimethamine.

Depending on the treatment protocol, the above composition of the kit, i.e., two or three dosage forms comprising the active ingredients, can be repeated in the composition kit according to the present invention such as for example a pharmaceutical composition kit comprising three times a tablet comprising artesunate and three times a tablet comprising sulfamethoxypyrazine and pyrimethamine, thereby providing the complete malarial treatment in one pharmaceutical composition kit.

According to the present invention, the pharmaceutical composition kit can be further provided with instructions for use, such as for example instructions for use in the form of pictograms depicting the necessary treatment protocol or in written form.

The pharmaceutical composition according to the present invention provides a treatment of malaria within one day through its active constituents. The pharmaceutical composition according to the present invention, or its active constituents, also provides the reoccurrence of the disease during at least a period of 28 days. In addition, also a reinfection with Plasmodium falciparum is prevented during this period providing in addition a prophylaxis for malaria.

Therefore, the present invention relates also to a combination of an antimalarial agent selected from the group consisting of artemether, arteether, artemisinin, dihydroartemisinin and artesunate; sulfamethoxypyrazine and a dihydrofolate reductase inhibitor or a pharmaceutical composition according to the present invention for use in the prophylaxis and/or treatment of malaria.

According to the present invention, the antimalarial agent is preferably artesunate and the dihydrofolate reductase inhibitor is preferably pyrimethamine.

As stated above, the pharmaceutical composition according to the present invention provides a treatment of malaria within one day through its active constituents, the reoccurrence of the disease during at least a period of 28 days and prevents a reinfection with Plasmodium falciparum during at least this period.

Therefore, the present invention also relates to the use of a combination of an antimalarial agent selected from the group consisting of artemether, arteether, artemisinin, dihydroartemisinin and artesunate; sulfamethoxypyrazine and a dihydrofolate reductase inhibitor or a pharmaceutical composition according to the present invention for the preparation of a medicament for the prophylaxis and/or treatment of malaria.

With respect to the above use, the antimalarial agent is preferably artesunate and the dihydrofolate reductase inhibitor is preferably pyrimethamine.

For an effective treatment using the pharmaceutical composition according to the present invention, preferably the use comprises administering a therapeutic effective dose of the combination or the pharmaceutical composition in three intervals in one day. Such therapeutic effective dose is preferably obtained by orally administering three times 100 mg artesunate, 250 mg sulfamethoxypyrazine and 12.5 mg pyrimethamine whereby the administrations are 12 hours apart, thus at the beginning of the treatment, approximately 12 hours later and 24 hours later.

For example, starting in the morning, the treatment protocol can comprise administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively, administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively when the patient goes to sleep, and administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively, when the patient again wakes up, thereby completely curing malaria.

The present invention will be further exemplified in the following examples illustratively demonstrating the efficacy of the invention.

### EXAMPLES

### Example 1: preparation of tablets comprising artesunate-sulfalene-pyrimethamine

**Table 1, dose composition**

| Name | Formula | Function |
|---|---|---|
| Sulfamethoxypyrazine | 250 mg | Active ingredient |
| Pyrimethamine | 12.5 mg | Active ingredient |
| Artesunate | 100 mg | Active ingredient |
| Maltodextrine | 30 mg | Binder, disintegrant |
| Microcrystalline cellulose | 168.1 mg | Filler |
| Colloidal silica anhydrous | 3.4 mg | Flow agent |
| Magnesium stearate | 6 mg | Lubricant |
| Total per tablet | 600 mg | |

### Manufacturing

Sulfamethoxypyrazine, pyrimethamine and maltodextrine were mixed in a granulator. Granulation was started by addition of 20 % (w/w) of water. After granulation and drying, the granulate was passed through a stainless steel sieve. Artesunate, Microcrystalline cellulose and Colloidal silica anhydrous were added and a blend was prepared in a mixer. Subsequently, Magnesium stearate was added and a final blend prepared. The resulting pre-compression mixture was used for tableting.

### Example 2: clinical trial: comparison between artesunate-sulfamethoxypyrazine-pyrimethamine and artemether-lumefantrine

In this example, the efficacy and safety of the combination artesunate-sulfamethoxypyrazine-pyrimethamine was tested against artemether-lumefantrine for the treatment of Plasmodium falciparum malaria.

### Methods

The study was carried out in a peri-urban area of the capital of an African nation, where malaria is mesoendemic and highly seasonal. Subjects aged ∃ 6 months with P. falciparum malaria were randomly assigned to treatment with artesunate-sulfamethoxypyrazine-pyrimethamine or artemether-lumefantrine. Treatment efficacy was assessed using the 28-day WHO 2003 protocol.

Treatment safety was assessed clinically as well as by measuring hematological indices and serum creatinine and hepatic transaminase concentrations. The sample size was computed assuming equal efficacy of the two treatments, and 606 patients (303 in each treatment arm) were enrolled over two consecutive malaria transmission seasons.

### Results

Baseline characteristics were similar between the two treatment groups. No cases of early treatment failure were observed. The cure rate after 28 days was higher for artesunate-sulfamethoxypyrazine-pyrimethamine (98.7%) than artemether-lumefantrine (89.6%), p < 0.0001. However, after correction for cases of re-infection, the 28-day cure rate was 100% for artesunate-sulfamethoxypyrazine-pyrimethamine, compared to 99.0% for artemether-lumefantrine (p=0.3).

Parasite and fever clearance were similar in the two treatments except that after 1 day, 90.8% of those in the artesunate-sulfamethoxypyrazine-pyrimethamine group were without fever versus 77.6% in the artemether-lumefantrine group (p < 0.001). Gametocyte carriage rates were similar in both treatment groups.

Anemia rate decreased significantly in artesunate-sulfamethoxypyrazine-pyrimethamine group after treatment as compared to before treatment (12.7 vs 6.5; p = 0.0003). No serious adverse events or significant laboratory abnormalities occurred.

In the artesunate-sulfamethoxypyrazine-pyrimethamine group, 2/82 (2.4%) and 1/87 (1.2%) as compared to 0/72 and 1/76 (1.3) in artemether-lumefantrine group had mild and transient elevation of transaminases and creatinine, respectively, on day 14 (p > 0.45).

The abnormality of hepatic transaminase and serum creatinine for these subjects resolved spontaneously within 2 weeks, confirming the safety of the pharmaceutical composition according to the present invention.

### Discussion

Administered as a single dose, artesunate-sulfamethoxypyrazine-pyrimethamine for three-days is at least as well-tolerated and effective as artemether-lumefantrine for the treatment of Plasmodium falciparum malaria.

Artesunate-sulfamethoxypyrazine-pyrimethamine cleared fever faster than artemether-lumefantrine on day 1. Artesunate-sulfamethoxypyrazine-pyrimethamine showed an additional benefit in the prevention of new infections during the 28-day period following treatment and reduced the anemia rates in comparison to artemether-lumefantrine.

### Example 3: Clinical trial: 24 hours treatment protocol versus a 72 hours treatment protocol using artesunate-sulfamethoxypyrazine-pyrimethamine

The efficacy of pharmaceutical compositions containing artesunate, sulfamethoxypyrazine and pyrimethamine was tested in two different treatment regimes.

Subjects aged ∃ 6 months with Plasmodium falciparum malaria were randomly assigned to two different treatment regimes with artesunate-sulfamethoxypyrazine-pyrimethamine. Either 3 doses over 3 days or 3 doses within 24 hours were administered. Treatment efficacy was assessed using the 28-day WHO 2003 protocol. Treatment safety was assessed clinically. The sample size was computed assuming equal efficacy of the two treatments leading to 200 patients (100 in each study arm).

### Results

**Table 2: Patient Baseline characteristics**

| | **24 hours protocol** | **72 hours protocol** |
|---|---|---|
| **number** | 100 | 100 |
| **male/female** | 59/41 | 49/51 |
| **age (years)** | 8.6 (1-43) | 9.6 (1-58) |
| **weight (kg)** | 27.6 (8-79) | 26.8 (6.5-78) |
| **Temperature (EC)** | 39.0 (37.5-41.0) | 38.7 (37.5-41.0) |
| **Parasitaemia** | 17.715 (2000-102640) | 22934 (1200-154000) |

The baseline characteristics of both groups were similar. Only people that could be followed up for 28 days were included.

**Table 3: temperature (EC) after treatment**

| | **24 hours protocol** | **72 hours protocol** |
|---|---|---|
| **day 1** | 37.4 (36.7 B 39.1) | 37.3 (34.4 - 38.5) |
| **day 2** | 37.2 (36.6 B 38.2) | 37.1 (34.2 - 38.0) |
| **day 3** | 37.0 (36.5 B 37.9) | 37.1 (36.5 B 37.7) |
| **day 7** | 37.1 (36.6 B 37.8) | 37.1 (36.5 B 39.0) |
| **day 14** | 37.0 (36.6 B 37.6) | 37.1 (36.5 B 38.0) |
| **day 21** | 37.1 (36.6 B 39.5) | 37.1 (36.6 B 38.7) |
| **day 28** | 37.0 (34.6 B 37.5) | 37.0 (36.5 B 37.6) |

**Table 4: Parasitaemia**

| | **24 hours protocol** | **72 hours protocol** |
|---|---|---|
| **day 1** | 0 | 11 (0-860)* |
| **day 2** | 0 | 0 |
| **day 3** | 0 | 0 |
| **day 7** | 0 | 0 |
| **day 14** | 0 | 0 |
| **day 21** | 0 | 0 |
| **day 28** | 0 | 0 |

| | | |
|---|---|---|
| *only 1 patient was found positive for parasites | | |

No serious adverse events or significant laboratory abnormalities occurred.

### Discussion

The data obtained show that the pharmaceutical composition according to the present invention, administered within 24 hours, is as least as efficacious and safe as the pharmaceutical composition according to the present invention administered over 3 days.

## Claims

1. Pharmaceutical composition suitable for the treatment of malaria in three intervals within one day comprising artesunate; sulfamethoxypyrazine and pyrimethamine and further comprising one or more pharmaceutical acceptable excipients, carriers and/or diluents wherein said pharmaceutical composition is in the form of a tablet.

2. Pharmaceutical composition according to claim 1, wherein the ratio between artesunate and sulfamethoxypyrazine is (50 to 150):(200 to 300), preferably 100:250.

3. Pharmaceutical composition according to claim 1 or claim 2, wherein the ratio between sulfamethoxypyrazine and pyrimethamine is (200 to 300):(10 to 15), preferably 250:12.5.

4. Pharmaceutical composition according to any of the claims 1 to 3, wherein the ratio between artesunate and pyrimethamine is (50 to 150):(10 to 15), preferably 100:12.5.

5. Pharmaceutical composition according to any of the claims 1 to 4 comprising 50 mg artesunate, 125 mg sulfamethoxypyrazine and 6.1 mg pyrimethamine for children; 100 mg artesunate, 250 mg sulfamethoxypyrazine and 12.5 mg pyrimethamine for youngsters; or 200 mg artesunate, 500 mg sulfamethoxypyrazine and 25 mg pyrimethamine for adults.

## Patentansprüche

1. Pharmazeutische Zusammensetzung geeignet zur Behandlung von Malaria in drei Zeiträumen innerhalb eines Tages umfassend Artesunat; Sulfamethoxypyridazin und Pyrimethamin und ferner umfassend einen oder mehrere pharmazeutisch annehmbare Hilfsstoffe, Träger und/oder Verdünnungsmittel, wobei die pharmazeutische Zusammensetzung in Form einer Tablette ist.

2. Pharmazeutische Zusammensetzung nach Anspruch 1, wobei das Verhältnis zwischen Artesunat und Sulfamethoxypyridazin (50 zu 150) : (200 zu 300) ist, bevorzugt 100:250.

3. Pharmazeutische Zusammensetzung nach Anspruch 1 oder Anspruch 2, wobei das Verhältnis zwischen Sulfamethoxypyridazin und Pyrimethamin (200 zu 300) : (10 zu 15) ist, bevorzugt 250:12,5.

4. Pharmazeutische Zusammensetzung nach einem der Ansprüche 1 bis 3, wobei das Verhältnis zwischen Artesunat und Pyrimethamin (50 zu 150):(10 zu 15) ist, bevorzugt 100:12,5.

5. Pharmazeutische Zusammensetzung nach einem der Ansprüche 1 bis 4 umfassend 50 mg Artesunat, 125 mg Sulfamethoxypyradizin und 6.1 mg Pyrimethamin für Kinder; 100 mg Artesunat, 250 mg Sulfamethoxypyradizin und 12.5 mg Pyrimethamin für Jugendliche; oder 200 mg Artesunat, 500 mg Sulfamethoxypyradizin und 25 mg Pyrimethamin für Erwachsene.

## Revendications

1. Composition pharmaceutique permettant le traitement du paludisme en trois intervalles sur une journée comprenant de l'artésunate, de la sulfaméthoxypyrazine et de la pyriméthamine, et comprenant en outre un ou plusieurs excipients, supports et/ou diluants pharmaceutiquement acceptables, ladite composition pharmaceutique se présentant sous la forme d'un comprimé.

2. Composition pharmaceutique selon la revendication 1, dans laquelle le rapport entre l'artésunate et la sulfaméthoxypyrazine est de (50 à 150):(200 à 300), de préférence de 100:250.

3. Composition pharmaceutique selon la revendication 1 ou 2, dans laquelle le rapport entre la sulfaméthoxypyrazine et la pyriméthamine est de (200 à 300):(10 à 15), de préférence de 250:12,5.

4. Composition pharmaceutique selon l'une quelconque des revendications 1 à 3, dans laquelle le rapport entre l'artésunate et la pyriméthamine est de (50 à 150):(10 à 15), de préférence de 100:12,5.

5. Composition pharmaceutique selon l'une quelconque des revendications 1 à 4, comprenant 50 mg d'artésunate, 125 mg de sulfaméthoxypyrazine et 6,1 mg de pyriméthamine pour les enfants ; 100 mg d'artésunate, 250 mg de sulfaméthoxypyrazine et 12,5 mg de pyriméthamine pour les adolescents ; ou 200 mg d'artésunate, 500 mg de sulfaméthoxypyrazine et 25 mg de pyriméthamine pour les adultes.
